# EUROPEAN PATENT APPLICATION

(11) **EP 4 850 744 A1**
(43) Date of publication of application: **09.12.2026**
(21) Application number: 26183214.1
(22) Date of filing: 05.06.2026
(51) Int. Cl.: A61M 60/13, A61M 60/174, A61M 60/221, A61M 60/515, A61M 60/523, A61M 60/531, A61M 60/562, A61M 60/569, A61M 60/585

(54) **SYSTEMS AND METHODS FOR CHANGING AN OPERATING MODE OF A BLOOD PUMP SYSTEM**

(30) Priority: 05.06.2025 US 202563818155 P
(71) Applicant: Abiomed, Inc., Danvers, MA 01923 (US)
(72) Inventor: KYROLLOS, Alfred, Danvers, 01923 (US); KIM, Jin Kwang, Danvers, MA, 01923 (US); CUCHIARA, Michael, Danvers, 01923 (US); UNUDURTHI, Sathya Dev, Danvers, 01923 (US)
(74) Representative: Wittmer, Maximilian

(57) **Abstract**

A blood pump system is described. The blood pump system includes a blood pump including a rotor, a motor configured to drive rotation of the rotor according to a first timing mode, and a controller. The controller is configured to receive a trigger signal indicating low blood flow through the blood pump, and control the motor to drive rotation of the rotor according to a second timing mode in response to receiving the trigger signal, wherein the second timing mode includes a delay relative to the first timing mode.

## Description

### BACKGROUND

Fluid pumps, such as blood pumps, are used in the medical field in a wide range of applications and purposes. An intravascular blood pump is a pump that can be advanced through a patient's vasculature, i.e., veins and/or arteries, to a position in the patient's heart or elsewhere within the patient's circulatory system. For example, an intravascular blood pump may be inserted via a catheter and positioned to span a heart valve. The intravascular blood pump is typically disposed at the end of the catheter. Once in position, the pump may be used to assist the heart and pump blood through the circulatory system and, therefore, temporarily reduce workload on the patient's heart, such as to enable the heart to recover after a heart attack. An exemplary intravascular blood pump is available from ABIOMED, Inc., Danvers, MA under the tradename Impella^{®} heart pump.

An intravascular blood pump is typically connected to a respective external heart pump controller that controls the heart pump, such as motor speed, and collects and displays operational data about the blood pump, such as heart signal level, battery temperature, blood flow rate and plumbing integrity. An exemplary heart pump controller is available from ABIOMED, Inc. under the trade name Automated Impella Controller^{®}. The controller may raise alarms when operational data values fall outside predetermined values or ranges, for example if a leak, suction, and/or pump malfunction is detected. The controller may include a video display screen upon which is displayed a graphical user interface configured to display the operational data and/or alarms.

### SUMMARY

In some embodiments, a blood pump system is provided. The blood pump system includes a blood pump including a rotor, a motor configured to drive rotation of the rotor according to a first timing mode, and a controller. The controller is configured to receive a trigger signal indicating low blood flow through the blood pump, and control the motor to drive rotation of the rotor according to a second timing mode in response to receiving the trigger signal, wherein the second timing mode includes a delay relative to the first timing mode.

In one aspect, the first timing mode is configured to drive rotation of the rotor in accordance with each cardiac cycle of a heart of a patient. In another aspect, the controller is further configured to detect a suction event associated with operation of the blood pump, and

the trigger signal is received in response to detecting the suction event. In another aspect, the trigger signal is received in response to determining that the suction event has persisted for an amount of time that exceeds a threshold amount of time. In another aspect, the controller is configured to generate the trigger signal based, at least in part, on one or more ventricular parameters. In another aspect, the one or more ventricular parameters include one or more of ventricular volume, ventricular pressure, or contractility. In another aspect, the controller is further configured to generate the trigger signal based, at least in part, on a current speed of the motor. In another aspect, the controller is further configured to receive the one or more ventricular parameters from a device separate from the blood pump system. In another aspect, the device separate from the blood pump system comprises one or more of an impedance catheter, a swan-ganz catheter, an imaging device, an echocardiography device, or an ultrasound patch. In another aspect, the controller is further configured to receive one or more signals from a device separate from the blood pump system, and determine the one or more ventricular parameters based on the received one or more signals.

In another aspect, the second timing mode is configured to drive rotation of the rotor at a multiple of a cardiac cycle of a heart of a patient. In another aspect, the second timing mode is configured to drive rotation of the rotor using a timing longer than a cardiac cycle of a heart of a patient. In another aspect, the controller is further configured to display a user interface, and the trigger signal is received via the user interface. In another aspect, the controller is further configured to receive feedback associated with blood flow through the blood pump when operated according to the second timing mode, and adjust the delay of the second timing mode based, at least in part, on the feedback. In another aspect, adjusting the delay of the second timing mode comprises increasing the delay.

In another aspect, the controller is further configured to receive feedback associated with blood flow through the blood pump when operated according to the second timing mode, and control the motor to drive rotation of the rotor according to the first timing mode based, at least in part, on the feedback. In another aspect, the feedback includes values for one or more ventricular parameters. In another aspect, the one or more ventricular parameters include one or more of ventricular volume, ventricular pressure, or contractility. In another aspect, the controller is further configured to control, after a predetermined amount of time following entering the second timing mode, the motor to drive rotation of the rotor according to the first timing mode, receive feedback associated with blood flow through the blood pump when operated according to the first timing mode, and control the motor to operate in the first timing mode or the second timing mode based, at least in part, on the feedback. In another aspect, the feedback includes values for one or more ventricular parameters. In another aspect, the one or more ventricular parameters include one or more of ventricular volume, ventricular pressure, or contractility.

In some embodiments, a method of operating a blood pump system is provided. The method includes controlling a motor of the blood pump system to operate according to a first timing mode, receiving a trigger signal indicating low blood flow through a blood pump of the blood pump system when the motor is operated according to the first timing mode, and controlling the motor to operate according to a second timing mode in response to receiving the trigger signal, wherein the second timing mode includes a delay relative to the first timing mode.

In one aspect, controlling the motor of the blood pump system to operate according to a first timing mode comprises controlling the motor to drive rotation of a rotor of the blood pump in accordance with each cardiac cycle of a heart of a patient. In another aspect, the method further includes detecting a suction event associated with operation of the blood pump, and the trigger signal is received in response to detecting the suction event. In another aspect, the trigger signal is received in response to determining that the suction event has persisted for an amount of time that exceeds a threshold amount of time. In another aspect, the method further includes generating the trigger signal based, at least in part, on one or more ventricular parameters. In another aspect, the one or more ventricular parameters include one or more of ventricular volume, ventricular pressure, or contractility. In another aspect, generating the trigger signal is further based, at least in part, on a current speed of the motor. In another aspect, the method further includes receiving the one or more ventricular parameters from a device separate from the blood pump system. In another aspect, the device separate from the blood pump system comprises one or more of an impedance catheter, a swan-ganz catheter, an imaging device, an echocardiography device, or an ultrasound patch. In another aspect, the method further includes receiving one or more signals from a device separate from the blood pump system, and determining the one or more ventricular parameters based on the received one or more signals.

In another aspect, controlling the motor of the blood pump system to operate according to a second timing mode comprises controlling the motor to drive rotation of a rotor of the blood pump at a multiple of a cardiac cycle of a heart of a patient. In another aspect, controlling the motor of the blood pump system to operate according to a second timing mode comprises controlling the motor to drive rotation of a rotor of the blood pump using a timing longer than a cardiac cycle of a heart of a patient. In another aspect, the method further includes displaying a user interface, and the trigger signal is received via the user interface. In another aspect, the method further includes receiving feedback associated with blood flow through the blood pump when operated according to the second timing mode, and adjusting the delay of the second timing mode based, at least in part, on the feedback. In another aspect, adjusting the delay of the second timing mode comprises increasing the delay.

In another aspect, the method further includes receiving feedback associated with blood flow through the blood pump when operated according to the second timing mode, and controlling the motor to drive rotation of a rotor of the blood pump according to the first timing mode based, at least in part, on the feedback. In another aspect, the feedback includes values for one or more ventricular parameters. In another aspect, the one or more ventricular parameters include one or more of ventricular volume, ventricular pressure, or contractility. In another aspect, the method further includes controlling, after a predetermined amount of time following entering the second timing mode, the motor to drive rotation of a rotor of the blood pump according to the first timing mode, receiving feedback associated with blood flow through the blood pump when operated according to the first timing mode, and controlling the motor to operate in the first timing mode or the second timing mode based, at least in part, on the feedback. In another aspect, the feedback includes values for one or more ventricular parameters. In another aspect, the one or more ventricular parameters include one or more of ventricular volume, ventricular pressure, or contractility.

In some embodiments, a controller for a blood pump system is provided. The controller includes at least one hardware processor configured to receive a trigger signal indicating low blood flow through a blood pump of the blood pump system when a motor of the blood pump system is operated according to a first timing mode, and control the motor to operate according to a second timing mode in response to receiving the trigger signal, wherein the second timing mode includes a delay relative to the first timing mode.

In one aspect, the first timing mode is configured to drive rotation of a rotor of the blood pump in accordance with each cardiac cycle of a heart of a patient. In another aspect, the at least one hardware processor is further configured to detect a suction event associated with operation of the blood pump, and the trigger signal is received in response to detecting the suction event. In another aspect, the trigger signal is received in response to determining that the suction event has persisted for an amount of time that exceeds a threshold amount of time. In another aspect, the at least one hardware processor is further configured to generate the trigger signal based, at least in part, on one or more ventricular parameters. In another aspect, the one or more ventricular parameters include one or more of ventricular volume, ventricular pressure, or contractility. In another aspect, the at least one hardware processor is further configured to generate the trigger signal based, at least in part, on a current speed of the motor. In another aspect, the at least one hardware processor is further configured to receive the one or more ventricular parameters from a device separate from the blood pump system. In another aspect, the device separate from the blood pump system comprises one or more of an impedance catheter, a swan-ganz catheter, an imaging device, an echocardiography device, or an ultrasound patch. In another aspect, the at least one hardware processor is further configured to receive one or more signals from a device separate from the blood pump system, and determine the one or more ventricular parameters based on the received one or more signals. In another aspect, the second timing mode is configured to drive rotation of a rotor of the blood pump at a multiple of a cardiac cycle of a heart of a patient. In another aspect, the second timing mode is configured to drive rotation of a rotor of the blood pump using a timing longer than a cardiac cycle of a heart of a patient. In another aspect, the at least one hardware processor is further configured to display a user interface, and the trigger signal is received via the user interface.

In another aspect, the at least one hardware processor is further configured to receive feedback associated with blood flow through the blood pump when operated according to the second timing mode, and adjust the delay of the second timing mode based, at least in part, on the feedback. In another aspect, adjusting the delay of the second timing mode comprises increasing the delay. In another aspect, the at least one hardware processor is further configured to receive feedback associated with blood flow through the blood pump when operated according to the second timing mode, and control the motor to drive rotation of a rotor of the blood pump according to the first timing mode based, at least in part, on the feedback. In another aspect, the feedback includes values for one or more ventricular parameters. In another aspect, the one or more ventricular parameters include one or more of ventricular volume, ventricular pressure, or contractility. In one aspect, the at least one hardware processor is further configured to control, after a predetermined amount of time following entering the second timing mode, the motor to drive rotation of a rotor of the blood pump according to the first timing mode, receive feedback associated with blood flow through the blood pump when operated according to the first timing mode, and control the motor to operate in the first timing mode or the second timing mode based, at least in part, on the feedback. In another aspect, the feedback includes values for one or more ventricular parameters. In another aspect, the one or more ventricular parameters include one or more of ventricular volume, ventricular pressure, or contractility.

### BRIEF DESCRIPTION OF DRAWINGS

FIG. 1A shows an illustrative cardiac support device that may be used with some embodiments.
FIG. 1B shows an illustrative cardiac support system that includes the cardiac support device of FIG. 1A.
FIG. 2 shows a schematic block diagram of a blood pump system according to some embodiments.
FIG. 3 shows a flowchart of a process according to some embodiments.

### DETAILED DESCRIPTION

Blood pumps may be implanted into a patient's heart to assist the heart and pump blood through the circulatory system, thereby temporarily reducing workload on the patient's heart, such as to enable the heart to recover after an acute injury (e.g., after a heart attack). When placed in the left side of the heart, the blood pump may configured to operate by pumping blood from a patient's left ventricle across the aortic valve and into the aorta with a timing that follows a patient's cardiac cycle to mimic (or approximate) native heart function. Timing the pump operation based on a patient's cardiac cycle may work well when there is sufficient blood in the left ventricle to pump across the aortic valve. However, when the left ventricle does not adequately fill with blood prior to operating the pump and/or there is some other event that causes low flow through the pump, only a small amount of blood may be pumped by the blood pump into the aorta. In some instances, inadequate filling of the ventricle may lead to a partial or complete obstruction of the flow through the pump, which may compromise pump function, resulting in reduced patient support. The obstructed flow may be due to ingestion of biomaterial into the pump leading to a permanent obstruction of the flow or a suction event leading to a reversable obstruction of the pump inlet. In the case of a suction event, a healthcare provider or other user may attempt to reposition the pump or take some other corrective action to improve the flow through the pump.

The inventors have recognized and appreciated that for some patients with low cardiac output (e.g., due to insufficient filling of the left ventricle, occurrence of a suction event, etc.), it may be advantageous to operate a blood pump according to a timing mode that introduces a delay between pumping cycles to allow the left ventricle more time to fill with blood prior to pumping a bolus of blood across the aortic valve. In some embodiments, a blood pump system may be configured to operate in at least two different timing modes including a first mode that is timed to the cardiac cycle of a patient and a second timing mode that introduces a delay between pumping cycles relative to the timing in the first timing mode. The delay between pumping cycles when the pump is operating in the second timing mode may allow for additional filling of the ventricle prior to a next pumping cycle to pump blood across the aortic valve.

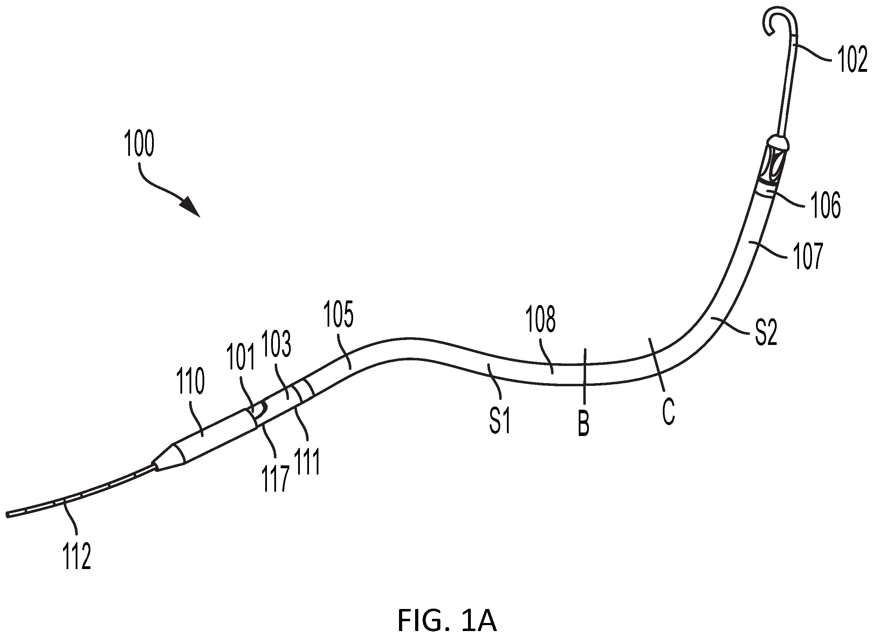

FIG. 1A shows an illustrative embodiment of a blood pump assembly 100 according to the present disclosure. The blood pump assembly 100 may include a pump 101, a pump housing 103, a proximal end 105, a distal end 107, a cannula 108, an impeller (not shown), an atraumatic extension 102, a catheter 112, an inlet area 110, an outlet area 106, and blood exhaust apertures 117. The catheter 112 may be connected to the inlet area 110 of the cannula 108 in some embodiments. The inlet area 110 may be located near the proximal end 105 of the cannula, and the outlet area 106 may be located toward the distal end 107 of the cannula 108. The inlet area 110 may include a pump housing 103 with a peripheral wall 111 extending about a rotation axis of the impeller blades, positioned radially outward of the inner surface with respect to the rotation axis of the impeller. The impeller may be rotatably coupled to the pump 101 at the inlet area 110 adjacent to the blood exhaust apertures 117 formed in the peripheral wall 111 of the pump housing 103. The pump housing 103 may be composed of a metal in accordance with some implementations. The atraumatic extension 102, also referred to as a "pigtail," may be connected to the distal end 107 of the cannula 108 and may assist with stabilizing and/or positioning the blood pump assembly 100 into the correct position in the heart. The atraumatic extension 102 may be configurable from a straight to a partially curved configuration. The atraumatic extension 102 may be composed, at least in part of a flexible material, and may have dual stiffness. It should be appreciated that some embodiments of the pump assembly may not include atraumatic extension 102.

The cannula 108 may have a shape which matches (or is similar to) the anatomy of the right ventricle of a patient. In the exemplary embodiment shown in FIG. 1A, the cannula has a proximal end 105 arranged to be located near the patient's inferior vena cava, and a distal end 107 arranged to be located near the pulmonary artery. The cannula 108 may include a first segment SI extending from the inflow area to a point B between the inlet area 110 and the outlet area 106. The cannula 108 may also include a second segment S2 extending from a point C, which is between the inlet area 110 and the outlet area 106, to the outlet area 106. In some implementations, points B and C may be located at the same location along cannula 108. The first segment SI of the cannula may form an 'S' shape in a first plane. In some implementations, segment SI can have curvatures between 30 degrees and 180 degrees. The second segment S2 of the cannula may form an 'S' shape in a second plane. In some implementations, segment S2 can have curvatures between 30 degrees and 180 degrees (e.g., 40°, 50°, 60°, 70°, 80°, 90°, 100°, 110°, 120°, 130°, 140°, 150°, 160°, or 170°). The second plane can be different from the first plane. In some implementations, the second plane may be parallel or identical to the first plane.

Although shown with an 'S' shape, it will be appreciated that other implementations of the blood pump assembly may be formed with other shapes (e.g., a 'U' shape), or with no shape at all when outside the body. In such implementations, the cannula may be formed of a flexible material such that the cannula may bend during insertion and achieve the desired shape once inside the heart of the patient.

In some implementations, the blood pump assembly 100 may be inserted percutaneously through the internal jugular vein, though the right atrium and into the right ventricle. When properly positioned, the blood pump assembly 100 may deliver blood from the inlet area 110, which sits inside the patient's right atrium, through the cannula 108, to the blood exhaust apertures 117 of the pump housing 103 positioned in the pulmonary artery. Alternatively, in some implementations the blood pump assembly 100 may be inserted percutaneously through the femoral artery and into the left ventricle to deliver blood from the left ventricle into the aorta.

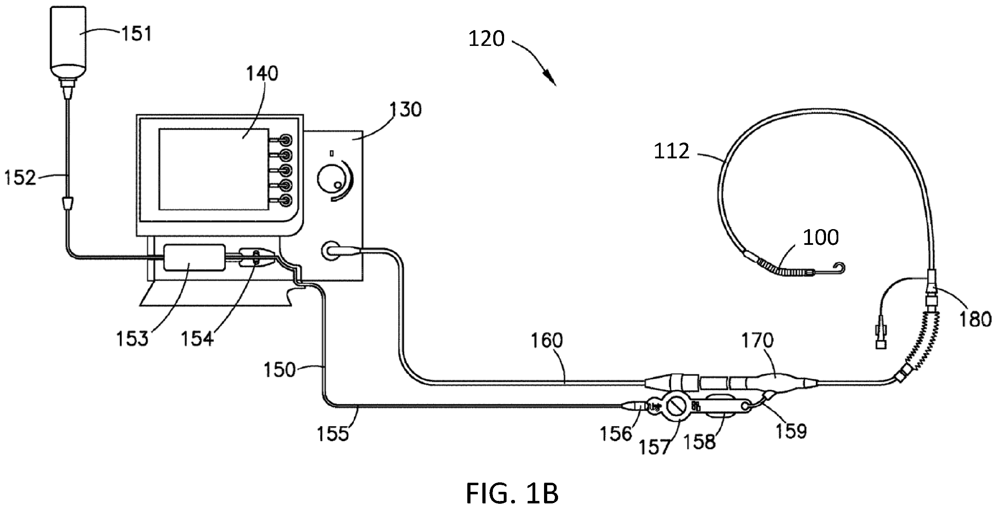

FIG. 1B shows that blood pump assembly 100 may form part of a cardiac support system 120. Cardiac support system 120 also may include a controller 130 (e.g., an Automated Impella Controller^{®}, referred to herein as an "AlC," from ABIOMED, Inc., Danvers, Mass.), a display 140, a purge subsystem 150, a connector cable 160, a plug 170, and a repositioning unit 180. As shown, controller 130 may include display 140. Controller 130 may be configured to monitor and control operation of blood pump assembly 100. During operation, purge subsystem 150 may be configured to deliver a purge fluid to blood pump assembly 100 through catheter 112 to prevent blood from entering the motor (not shown) of the blood pump. In some implementations, the purge fluid is a dextrose solution (e.g., 5% dextrose in water with 25 or 50 IU/mL of heparin, although the solution need not include heparin in all embodiments). Connector cable 160 may provide an electrical connection between blood pump assembly 100 and controller 130. Plug 170 may connect catheter 112, purge subsystem 150, and connector cable 160. In some implementations, plug 170 includes a storage device (e.g., a memory) configured to store, for example, operating parameters to facilitate transfer of the patient to another controller if needed. Repositioning unit 180 may be used to reposition blood pump assembly 100 in the patient's heart (e.g., by holding a position of the pump assembly relative to the patient).

As shown in FIG. 1B, in some embodiments, the cardiac support system 120 may include a purge subsystem 150 having a container 151, a supply line 152, a purge cassette 153, a purge disc 154, purge tubing 155, a check valve 156, a pressure reservoir 157, an infusion filter 158, and a sidearm 159. Container 151 may, for example, be a bag or a bottle. As will be appreciated, in other embodiments the cardiac support system 120 may not include a purge subsystem. In some embodiments, a purge fluid may be stored in container 151. Supply line 152 may provide a fluidic connection between container 151 and purge cassette 153. Purge cassette 153 may control how the purge fluid in container 151 is delivered to blood pump assembly 100. For example, purge cassette 153 may include one or more valves for controlling a pressure and/or flow rate of the purge fluid. Purge disc 154 may include one or more pressure and/or flow sensors for measuring a pressure and/or flow rate of the purge fluid. As shown, controller 130 may include purge cassette 153 and purge disc 154. Purge tubing 155 may provide a fluidic connection between purge disc 154 and check valve 156. Pressure reservoir 157 may provide additional filling volume during a purge fluid change. In some implementations, pressure reservoir 157 may include a flexible rubber diaphragm that provides the additional filling volume by means of an expansion chamber. Infusion filter 158 may help prevent bacterial contamination and air from entering catheter 112. Sidearm 159 may provide a fluidic connection between infusion filter 158 and plug 170. Although shown as having separate purge tubing and connector cable, it will be appreciated that in some embodiments, the cardiac support system 120 may include a single connector with both fluidic and electric lines connectable to the controller 130.

During operation, controller 130 may be configured to control the electrical power delivered to the motor of the blood pump assembly 100 (e.g., via connector cable 160), thereby controlling the speed of the motor to drive rotation of a rotor (not shown) of the blood pump assembly 100. Different motor speeds may be referred to herein as different "P-levels" with a P-level of P-1 corresponding to a minimum speed of the motor and a P-level of P-9 corresponding to a maximum speed of the motor. Blood pump assembly 100 may include a current sensor (not shown) configured sense motor current associated with an operating state of the motor, and controller 130 may be configured to receive the output of the current sensor as a motor current signal. Controller 130 may be configured to detect an obstruction event based, at least in part, on the motor current signal and a pump speed signal based on a measured speed of the pump, as described in more detail herein. The current sensor may be included in controller 130 or may be located along any portion of the connector cable 160 between controller 130 and the motor. Additionally or alternatively, the current sensor may be included in the motor and controller 130 may be configured to receive the motor current signal via a data line coupled to controller 130 and the motor.

In some embodiments, controller 130 may be configured to receive measurements from one or more pressure sensors (not shown) included as a portion of blood pump assembly 100 and purge disc 154. Controller 130 may be configured to control and measure a pressure and/or flow rate of a purge fluid via purge cassette 153 and purge disc 154. During operation, after exiting purge subsystem 150 through sidearm 159, the purge fluid may be channeled through purge lumens (not shown) within catheter 112 and plug 170. Sensor cables (not shown) within catheter 112, connector cable 160, and plug 170 may provide an electrical connection between components of the blood pump assembly 100 (e.g., one or more pressure sensors) and controller 130. Motor cables (not shown) within catheter 112, connector cable 160, and plug 170 may provide an electrical connection between the motor of the blood pump assembly 100 and controller 130. During operation, controller 130 may be configured to receive measurements from one or more pressure sensors of the blood pump assembly 100 through the sensor cables (e.g., optical fibers) and to control the electrical power delivered to the motor of the blood pump assembly 100 through the motor cables. By controlling the power delivered to the motor of the blood pump assembly 100, controller 130 may be operable to control the speed of the motor.

Various modifications can be made to cardiac support system 120 and one or more of its components. For instance, one or more additional sensors may be added to blood pump assembly 100. In another example, a signal generator may be added to blood pump assembly 100 to generate a signal indicative of the rotational speed of the motor of the blood pump assembly 100. As another example, one or more components of cardiac support system 120 may be separated. For instance, display 140 may be incorporated into another device in communication with controller 130 (e.g., wirelessly or through one or more electrical cables).

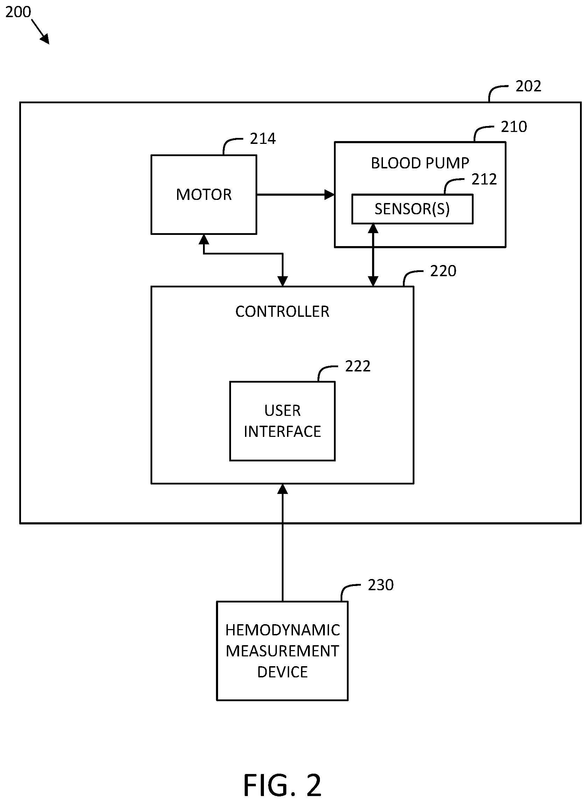

FIG. 2 illustrates a block diagram of a cardiac support system 200, which may include components similar to cardiac support system 120 shown in FIG. 1B. Cardiac support system 200 may include a blood pump system 202, which may include a blood pump 210, a motor 214 and a controller 220. Blood pump 210 may include one or more sensors 212 configured to sense physiological information associated with a heart of a patient within which blood pump 210 is placed. For example, in some embodiments, sensor(s) 212 may include one or more pressure sensors configured to sense a pressure inside of the heart of the patient. As shown in FIG. 2, data sensed by sensor(s) 212 may be provided to controller 220. Controller 220 may be configured to control operation of motor 214 to operate blood pump 210 at a particular speed and/or according to a particular timing mode. As described herein, in some embodiments, controller 220 may be configured to control operation of motor 214 according to a first timing mode and a second timing mode, wherein in the first timing mode the motor 214 is controlled to operate according to a cardiac cycle of a patient, and in the second timing mode the motor 214 is controlled to operate with a delay relative to the first timing mode. The delay in the second timing mode may enable additional filling of the left ventricle of the patient prior to pumping blood from the left ventricle of the aorta.

In some embodiments, controller 220 may be configured to present a user interface 222 on a display (e.g., a display associated with the controller and/or a remote display communicatively coupled to the controller via one or more network). A user may interact with user interface 222 to control operation of motor 214. For example, as described herein, a user may interact with user interface 222 to instruct controller to operate motor in accordance with a timing mode that permits additional filling of the left ventricle of a patient prior to a next pumping cycle. In some embodiments, controller 220 may be configured to receive input from one or more hemodynamic measurement devices 230 external to blood pump system 202. For instance, hemodynamic measurement device(s) 230 may include one or more devices configured to determine one or more ventricular parameters (e.g., ventricular volume, ventricular pressure, ventricular contractility, etc.). Examples of hemodynamics measurement device(s) 230 include, but are not limited to, an impedance catheter, a swan-ganz catheter, an imaging device (e.g., an ultrasound patch, a computed tomography device), an echocardiography device, or a pressure wire configured to directly measure a pressure inside of a patient's heart. As described herein controller 220 may be configured to use information received from sensor(s) 212, user interface 222 and/or hemodynamic measurement device(s) 230 to determine whether and/or how to control operation of motor 214.

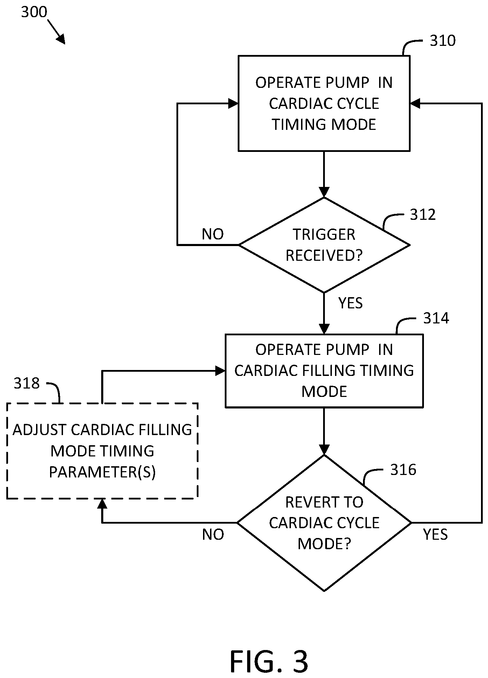

FIG. 3 illustrates a flowchart of a process 300 for operating a blood pump system, in accordance with some embodiments of the present disclosure. Process 300 may begin in act 310, where the blood pump system (e.g., blood pump system 202) is operated in a first timing mode based on a patient's cardiac cycle. In some embodiments, the first timing mode may be a default timing mode of the blood pump system in which the blood pump is configured to pump blood across the aortic valve with a pumping cycle that mimics (or approximates) the patient's native cardiac function. Process 300 may then proceed to act 312, where it may be determined whether a trigger signal has been received that indicates low blood flow through the blood pump when the blood pump system is operating in the first timing mode.

The trigger signal may be received and/or determined based on data from any suitable source. In some embodiments, the trigger signal may be received and/or determined based on data from one or more sensors (e.g., sensor(s) 212) associated with the blood pump system. For example, the blood pump system may include a blood pump having disposed thereon, one or more pressure sensors (e.g., one or more optical pressure sensors) configured to sense a pressure in a portion of a patient's heart (e.g., the left ventricular pressure, the aortic pressure). In some embodiments, sensor data from the one or more sensors may be provided to a controller (e.g., controller 220), and the controller may be configured to determine one or more ventricular parameters based, at least in part, on the sensor data. The one or more ventricular parameters may be used, at least in part, to generate the trigger signal received in act 312.

In some embodiments, a controller of the blood pump system may be configured to detect one or more events associated with operation of the blood pump system using data from one or more sensors (e.g., pressure sensor(s), motor current sensors, etc.), and the trigger signal may be generated based, at least in part, on the detection of the event(s). For instance, the controller may be configured to detect a suction event associated with operation of the blood pump system, and the trigger signal may be generated and/or received in response to detecting the suction event. In some embodiments, the trigger signal may be generated and/or received in response to detecting that a suction event has persisted for a threshold amount of time.

In some embodiments, the controller may be configured to generate the trigger signal based, at least in part, on a current speed of the motor of the blood pump system. For example, in some embodiments, the trigger signal may be generated only if the current speed of the motor is less than a threshold pump speed. In some embodiments, one or more different thresholds may be selected depending on the current speed of the motor, and the trigger signal may be generated, at least in part, on the selected threshold(s). For instance, a lower threshold for a ventricular parameter may be used when the motor is operating at a higher speed (e.g., P3), and a higher threshold for the ventricular parameter may be used to when the motor is operating at a lower speed (e.g., P1).

In some embodiments, the trigger signal may be received via a user interface associated with a controller of the blood pump system. For instance, a user may interact with the user interface to instruct the blood pump system to enter the second timing mode, and the trigger signal may be generated in response to the user instruction via the user interface. In some embodiments, the trigger signal may be received and/or determined from data received from one or more hemodynamic measurement devices. For example, a hemodynamics measurement device may be configured to directly measure a ventricular parameter, such as ventricular volume or ventricular pressure. In such an instance, the trigger signal may be generated when the ventricular parameter is less than a threshold value. In some embodiments, one or more ventricular parameters may be determined based, at least in part, on data received from one or more hemodynamic measurement devices, and the trigger signal may be generated based, at least in part, on the or more determined ventricular parameters.

Returning to process 300, if it is determined in act 312 that no trigger signal has been received, process 300 may return to act 310, where the blood pump system may continue to operate according to the first timing mode. If it is determined in act 312 that a trigger signal has been received, process 300 may proceed to act 314, where the blood pump system is operated in a second time mode, where the second timing mode includes a delay between pumping cycles relative to the first timing mode. In some embodiments, the second timing mode may be referred to as a "cardiac filling mode," in which the delay between pumping cycles of the blood pump may enable the left ventricle of the patient to fill more completely with blood prior to a next pumping cycle. The second timing mode may include any suitable delay between pumping cycles to allow for filling of the left ventricle. In some embodiments, the delay may be a multiple of the patient's cardiac cycle (e.g., two cardiac cycles, five cardiac cycles, ten cardiac cycles, etc.). In other embodiments, the delay may be a fixed amount of time (e.g., two seconds, five seconds, ten seconds, etc.), where the fixed amount of time is longer than a cardiac cycle of the heart of the patient.

Returning to process 300, after the blood pump system is controlled to operate in the second timing mode in act 314, process 300 proceeds to act 316, where it is determined whether to revert operation of the blood pump system to the first timing mode (e.g., the default timing mode). The determination of whether to revert operation of the blood pump system to the first timing mode may be made in any suitable way. For example, in some embodiments, data associated with one or more ventricular parameters (e.g., ventricular volume, ventricular pressure, contractility) may be measured and/or determined based on one or more measured signals, and it may be determined to revert operation to the first timing mode based, at least in part, on the one or more ventricular parameters. In some embodiments, a determination may be made to revert operation of the blood pump system to the first timing mode after a predetermined amount of time (e.g., 1 minute, 5 minutes, 10 minutes, 20 minutes, 1 hour, etc.) has passed since the blood pump system entered the second timing mode. In some embodiments, the controller may receive feedback associated with blood flow through the blood pump when the blood pump system is operating in the second timing mode, and the determination to revert operation of the blood pump system to the first timing mode may be made based, at least in part, on the feedback. For example, the feedback may include values for one or more ventricular parameters (e.g., ventricular volume, ventricular pressure, contractility, etc.) measured directly or indirectly by one or more sensors or devices. In some embodiments, the decision to revert operation of the blood pump system to the first timing mode may be based on a user input received via the user interface associated with the controller of the blood pump system. For instance, a user may interact with the user interface to instruct the blood pump system to enter the first (e.g., default) timing mode of operation.

If it is determined in act 316 that the blood pump system should revert operation of the blood pump system to the first timing mode, process 300 may return to act 310, where the blood pump system is controlled to operate in the first timing mode. If, after reverting operation of the blood pump system to the first timing mode, a trigger signal is received again in act 312, the blood pump system may again be controlled to operate in the second timing mode in act 314 with the same or different timing parameters as a previous time when the blood pump system was operating in the second timing mode. For instance, in some embodiments when the trigger signal is received within a threshold amount of time after operation is reverted to the first timing mode, the blood pump system may be controlled to operate according to the second timing mode, but with an increased delay relative to a previous time in which the blood pump system was operating in the second timing mode.

If it is determined in act 316 not to revert operation of the blood pump system to the first timing mode, process 300 may proceed to act 318 where one or more timing parameters of the second timing mode may optionally be adjusted. For instance, the delay included in the second timing mode may optionally be increased or decreased based on sensor data, user input, one or more signals received from a hemodynamic measurement device, or any combination thereof. In some embodiments, the controller may receive feedback associated with blood flow through the blood pump when the blood pump system is operating in the second timing mode, and the determination of whether to adjust one or more timing parameters of the second timing mode may be made based, at least in part, on the feedback. For example, the feedback may include values for one or more ventricular parameters (e.g., ventricular volume, ventricular pressure, contractility, etc.) measured directly or indirectly by one or more sensors or devices.

Having thus described several aspects and embodiments of the technology set forth in the disclosure, it is to be appreciated that various alterations, modifications, and improvements will readily occur to those skilled in the art. Such alterations, modifications, and improvements are intended to be within the spirit and scope of the technology described herein. For example, those of ordinary skill in the art will readily envision a variety of other means and/or structures for performing the function and/or obtaining the results and/or one or more of the advantages described herein, and each of such variations and/or modifications is deemed to be within the scope of the embodiments described herein. Those skilled in the art will recognize or be able to ascertain using no more than routine experimentation, many equivalents to the specific embodiments described herein. It is, therefore, to be understood that the foregoing embodiments are presented by way of example only and that, within the scope of the appended claims and equivalents thereto, inventive embodiments may be practiced otherwise than as specifically described. In addition, any combination of two or more features, systems, articles, materials, kits, and/or methods described herein, if such features, systems, articles, materials, kits, and/or methods are not mutually inconsistent, is included within the scope of the present disclosure.

The above-described embodiments can be implemented in any of numerous ways. One or more aspects and embodiments of the present disclosure involving the performance of processes or methods may utilize program instructions executable by a device (e.g., a computer, a processor, or other device) to perform, or control performance of, the processes or methods. In this respect, various inventive concepts may be embodied as a computer readable storage medium (or multiple computer readable storage media) (e.g., a computer memory, one or more floppy discs, compact discs, optical discs, magnetic tapes, flash memories, circuit configurations in Field Programmable Gate Arrays or other semiconductor devices, or other tangible computer storage medium) encoded with one or more programs that, when executed on one or more computers or other processors, perform methods that implement one or more of the various embodiments described above. The computer readable medium or media can be transportable, such that the program or programs stored thereon can be loaded onto one or more different computers or other processors to implement various ones of the aspects described above. In some embodiments, computer readable media may be non-transitory media.

The above-described embodiments of the present technology can be implemented in any of numerous ways. For example, the embodiments may be implemented using hardware, software or a combination thereof. When implemented in software, the software code can be executed on any suitable processor or collection of processors, whether provided in a single computer or distributed among multiple computers. It should be appreciated that any component or collection of components that perform the functions described above can be generically considered as a controller that controls the above-described function. A controller can be implemented in numerous ways, such as with dedicated hardware, or with general purpose hardware (e.g., one or more processor) that is programmed using microcode or software to perform the functions recited above and may be implemented in a combination of ways when the controller corresponds to multiple components of a system.

Further, it should be appreciated that a computer may be embodied in any of a number of forms, such as a rack-mounted computer, a desktop computer, a laptop computer, or a tablet computer, as non-limiting examples. Additionally, a computer may be embedded in a device not generally regarded as a computer but with suitable processing capabilities, including a Personal Digital Assistant (PDA), a smartphone or any other suitable portable or fixed electronic device.

Also, a computer may have one or more input and output devices. These devices can be used, among other things, to present a user interface. Examples of output devices that can be used to provide a user interface include printers or display screens for visual presentation of output and speakers or other sound generating devices for audible presentation of output. Examples of input devices that can be used for a user interface include keyboards, and pointing devices, such as mice, touch pads, and digitizing tablets. As another example, a computer may receive input information through speech recognition or in other audible formats.

Such computers may be interconnected by one or more networks in any suitable form, including a local area network or a wide area network, such as an enterprise network, and intelligent network (IN) or the Internet. Such networks may be based on any suitable technology and may operate according to any suitable protocol and may include wireless networks, wired networks or fiber optic networks.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

### EXEMPLARY EMBODIMENTS

A1. A blood pump system, comprising: a blood pump including a rotor; a motor configured to drive rotation of the rotor according to a first timing mode; and a controller configured to: receive a trigger signal indicating low blood flow through the blood pump; and control the motor to drive rotation of the rotor according to a second timing mode in response to receiving the trigger signal, wherein the second timing mode includes a delay relative to the first timing mode.
A2. The blood pump system of paragraph A1, wherein the first timing mode is configured to drive rotation of the rotor in accordance with each cardiac cycle of a heart of a patient.
A3. The blood pump system of paragraph A1 or A2, wherein the controller is further configured to detect a suction event associated with operation of the blood pump, and the trigger signal is received in response to detecting the suction event.
A4. The blood pump system of paragraph A3, wherein the trigger signal is received in response to determining that the suction event has persisted for an amount of time that exceeds a threshold amount of time.
A5. The blood pump system of any one of the preceding paragraphs A1 to A4, wherein the controller is configured to generate the trigger signal based, at least in part, on one or more ventricular parameters.
A6. The blood pump system of paragraph A5, wherein the one or more ventricular parameters include one or more of ventricular volume, ventricular pressure, or contractility.
A7. The blood pump system of paragraph A5 or A6, wherein the controller is further configured to generate the trigger signal based, at least in part, on a current speed of the motor.
A8. The blood pump system of any one of the preceding paragraphs A5 to A7, wherein the controller is further configured to receive the one or more ventricular parameters from a device separate from the blood pump system.
A9. The blood pump system of paragraph A8, wherein the device separate from the blood pump system comprises one or more of an impedance catheter, a swan-ganz catheter, an imaging device, an echocardiography device, or an ultrasound patch.
A10. The blood pump system of any one of the preceding paragraphs A5 to A9, wherein the controller is further configured to: receive one or more signals from a device separate from the blood pump system; and determine the one or more ventricular parameters based on the received one or more signals.
A11. The blood pump system of any one of the preceding paragraphs A1 to A10, wherein the second timing mode is configured to drive rotation of the rotor at a multiple of a cardiac cycle of a heart of a patient.
A12. The blood pump system of any one of the preceding paragraphs A1 to A11, wherein the second timing mode is configured to drive rotation of the rotor using a timing longer than a cardiac cycle of a heart of a patient.
A13. The blood pump system of any one of the preceding paragraphs A1 to A12, wherein the controller is further configured to display a user interface, and the trigger signal is received via the user interface.
A14. The blood pump system of any one of the preceding paragraphs A1 to A13, wherein the controller is further configured to: receive feedback associated with blood flow through the blood pump when operated according to the second timing mode; and adjust the delay of the second timing mode based, at least in part, on the feedback.
A15. The blood pump system of paragraph A14, wherein adjusting the delay of the second timing mode comprises increasing the delay.
A16. The blood pump system of any one of the preceding paragraphs A1 to A15, wherein the controller is further configured to: receive feedback associated with blood flow through the blood pump when operated according to the second timing mode; and control the motor to drive rotation of the rotor according to the first timing mode based, at least in part, on the feedback.
A17. The blood pump system of paragraph A16, wherein the feedback includes values for one or more ventricular parameters.
A18. The blood pump system of paragraph A17, wherein the one or more ventricular parameters include one or more of ventricular volume, ventricular pressure, or contractility.
A19. The blood pump system of any one of the preceding paragraphs A1 to A18, where the controller is further configured to: control, after a predetermined amount of time following entering the second timing mode, the motor to drive rotation of the rotor according to the first timing mode; receive feedback associated with blood flow through the blood pump when operated according to the first timing mode; and control the motor to operate in the first timing mode or the second timing mode based, at least in part, on the feedback.
A20. The blood pump system of paragraph A19, wherein the feedback includes values for one or more ventricular parameters.
A21. The blood pump system of paragraph A20, wherein the one or more ventricular parameters include one or more of ventricular volume, ventricular pressure, or contractility.
A22. A method of operating a blood pump system, the method comprising: controlling a motor of the blood pump system to operate according to a first timing mode; receiving a trigger signal indicating low blood flow through a blood pump of the blood pump system when the motor is operated according to the first timing mode; and controlling the motor to operate according to a second timing mode in response to receiving the trigger signal, wherein the second timing mode includes a delay relative to the first timing mode.
A23. The method of paragraph A22, wherein controlling the motor of the blood pump system to operate according to a first timing mode comprises controlling the motor to drive rotation of a rotor of the blood pump in accordance with each cardiac cycle of a heart of a patient.
A24. The method of paragraph A22 or A23, further comprising: detecting a suction event associated with operation of the blood pump, wherein the trigger signal is received in response to detecting the suction event.
A25. The method of paragraph A24, wherein the trigger signal is received in response to determining that the suction event has persisted for an amount of time that exceeds a threshold amount of time.
A26. The method of any one of the preceding paragraphs A22 to A25, further comprising generating the trigger signal based, at least in part, on one or more ventricular parameters.
A27. The method of paragraph A26, wherein the one or more ventricular parameters include one or more of ventricular volume, ventricular pressure, or contractility.
A28. The method of paragraph A26 or A27, wherein generating the trigger signal is further based, at least in part, on a current speed of the motor.
A29. The method of any one of the preceding paragraphs A26 to A28, further comprising receiving the one or more ventricular parameters from a device separate from the blood pump system.
A30. The method of paragraph A29, wherein the device separate from the blood pump system comprises one or more of an impedance catheter, a swan-ganz catheter, an imaging device, an echocardiography device, or an ultrasound patch.
A31. The method of any one of the preceding paragraphs A26 to A30, further comprising: receiving one or more signals from a device separate from the blood pump system; and determining the one or more ventricular parameters based on the received one or more signals.
A32. The method of any one of the preceding paragraphs A22 to A31, wherein controlling the motor of the blood pump system to operate according to a second timing mode comprises controlling the motor to drive rotation of a rotor of the blood pump at a multiple of a cardiac cycle of a heart of a patient.
A33. The method of any one of the preceding paragraphs A22 to A32, wherein controlling the motor of the blood pump system to operate according to a second timing mode comprises controlling the motor to drive rotation of a rotor of the blood pump using a timing longer than a cardiac cycle of a heart of a patient.
A34. The method of any one of the preceding paragraphs A22 to A33, further comprising: displaying a user interface, wherein the trigger signal is received via the user interface.
A35. The method of any one of the preceding paragraphs A22 to A34, further comprising:receiving feedback associated with blood flow through the blood pump when operated according to the second timing mode; and adjusting the delay of the second timing mode based, at least in part, on the feedback.
A36. The method of paragraph A35, wherein adjusting the delay of the second timing mode comprises increasing the delay.
A37. The method of any one of the preceding paragraphs A22 to A36, further comprising: receiving feedback associated with blood flow through the blood pump when operated according to the second timing mode; and controlling the motor to drive rotation of a rotor of the blood pump according to the first timing mode based, at least in part, on the feedback.
A38. The method of paragraph A37, wherein the feedback includes values for one or more ventricular parameters.
A39. The method of paragraph A38, wherein the one or more ventricular parameters include one or more of ventricular volume, ventricular pressure, or contractility.
A40. The method of any one of the preceding paragraphs A22 to A39, further comprising: controlling, after a predetermined amount of time following entering the second timing mode, the motor to drive rotation of a rotor of the blood pump according to the first timing mode; receiving feedback associated with blood flow through the blood pump when operated according to the first timing mode; and controlling the motor to operate in the first timing mode or the second timing mode based, at least in part, on the feedback.
A41. The method of paragraph A40, wherein the feedback includes values for one or more ventricular parameters.
A42. The method of paragraph A41, wherein the one or more ventricular parameters include one or more of ventricular volume, ventricular pressure, or contractility.
A43. A controller for a blood pump system, the controller comprising: at least one hardware processor configured to: receive a trigger signal indicating low blood flow through a blood pump of the blood pump system when a motor of the blood pump system is operated according to a first timing mode; and control the motor to operate according to a second timing mode in response to receiving the trigger signal, wherein the second timing mode includes a delay relative to the first timing mode.
A44. The controller of paragraph A43, wherein the first timing mode is configured to drive rotation of a rotor of the blood pump in accordance with each cardiac cycle of a heart of a patient.
A45. The controller of paragraph A43 or A44, wherein the at least one hardware processor is further configured to detect a suction event associated with operation of the blood pump, and the trigger signal is received in response to detecting the suction event.
A46. The controller of paragraph A45, wherein the trigger signal is received in response to determining that the suction event has persisted for an amount of time that exceeds a threshold amount of time.
A47. The controller of any one of the preceding paragraphs A43 to A46, wherein the at least one hardware processor is further configured to generate the trigger signal based, at least in part, on one or more ventricular parameters.
A48. The controller of paragraph A47, wherein the one or more ventricular parameters include one or more of ventricular volume, ventricular pressure, or contractility.
A49. The controller of paragraph A47 or A48, wherein the at least one hardware processor is further configured to generate the trigger signal based, at least in part, on a current speed of the motor.
A50. The controller of any one of the preceding paragraphs A47 to A49, wherein the at least one hardware processor is further configured to receive the one or more ventricular parameters from a device separate from the blood pump system.
A51. The controller of paragraph A50, wherein the device separate from the blood pump system comprises one or more of an impedance catheter, a swan-ganz catheter, an imaging device, an echocardiography device, or an ultrasound patch.
A52. The controller of any one of the preceding paragraphs A47 to A51, wherein the at least one hardware processor is further configured to: receive one or more signals from a device separate from the blood pump system; and determine the one or more ventricular parameters based on the received one or more signals.
A53. The controller of any one of the preceding paragraphs A43 to A52, wherein the second timing mode is configured to drive rotation of a rotor of the blood pump at a multiple of a cardiac cycle of a heart of a patient.
A54. The controller of any one of the preceding paragraphs A43 to A53, wherein the second timing mode is configured to drive rotation of a rotor of the blood pump using a timing longer than a cardiac cycle of a heart of a patient.
A55. The controller of any one of the preceding paragraphs A43 to A54, wherein the at least one hardware processor is further configured to display a user interface, and the trigger signal is received via the user interface.
A56. The controller of any one of the preceding paragraphs A43 to A55, wherein the at least one hardware processor is further configured to: receive feedback associated with blood flow through the blood pump when operated according to the second timing mode; and adjust the delay of the second timing mode based, at least in part, on the feedback.
A57. The controller of paragraph A56, wherein adjusting the delay of the second timing mode comprises increasing the delay.
A58. The controller of any one of the preceding paragraphs A43 to A57, wherein the at least one hardware processor is further configured to: receive feedback associated with blood flow through the blood pump when operated according to the second timing mode; and control the motor to drive rotation of a rotor of the blood pump according to the first timing mode based, at least in part, on the feedback.
A59. The controller of paragraph A58, wherein the feedback includes values for one or more ventricular parameters.
A60. The controller of paragraph A59, wherein the one or more ventricular parameters include one or more of ventricular volume, ventricular pressure, or contractility.
A61. The controller of any one of the preceding paragraphs A43 to A60, where the at least one hardware processor is further configured to: control, after a predetermined amount of time following entering the second timing mode, the motor to drive rotation of a rotor of the blood pump according to the first timing mode; receive feedback associated with blood flow through the blood pump when operated according to the first timing mode; and control the motor to operate in the first timing mode or the second timing mode based, at least in part, on the feedback.
A62. The controller of paragraph A61, wherein the feedback includes values for one or more ventricular parameters.
A63. The controller of paragraph A62, wherein the one or more ventricular parameters include one or more of ventricular volume, ventricular pressure, or contractility.

## Claims

1. A blood pump system, comprising:
a blood pump including a rotor;
a motor configured to drive rotation of the rotor according to a first timing mode; and
a controller configured to:
receive a trigger signal indicating low blood flow through the blood pump; and
control the motor to drive rotation of the rotor according to a second timing mode in response to receiving the trigger signal, wherein the second timing mode includes a delay relative to the first timing mode.

2. The blood pump system of claim 1, wherein the first timing mode is configured to drive rotation of the rotor in accordance with each cardiac cycle of a heart of a patient, and/ or
wherein the controller is further configured to detect a suction event associated with operation of the blood pump, and
the trigger signal is received in response to detecting the suction event, preferably, wherein the trigger signal is received in response to determining that the suction event has persisted for an amount of time that exceeds a threshold amount of time.

3. The blood pump system of claim 1 or 2, wherein the controller is configured to generate the trigger signal based, at least in part, on one or more ventricular parameters,
wherein the one or more ventricular parameters preferably include one or more of ventricular volume, ventricular pressure, or contractility, and/ or
wherein the controller is preferably further configured to generate the trigger signal based, at least in part, on a current speed of the motor, and/ or
wherein the controller is preferably further configured to receive one or more signals from a device separate from the blood pump system; and determine the one or more ventricular parameters based on the received one or more signals, and/ orwherein the controller is preferably further configured to receive the one or more ventricular parameters from a device separate from the blood pump system, wherein the device separate from the blood pump system preferably comprises one or more of an impedance catheter, a swan-ganz catheter, an imaging device, an echocardiography device, or an ultrasound patch.

4. The blood pump system of any one of the preceding claims, wherein the second timing mode is configured to drive rotation of the rotor at a multiple of a cardiac cycle of a heart of a patient, and/ or wherein the second timing mode is configured to drive rotation of the rotor using a timing longer than a cardiac cycle of a heart of a patient, and/ or
wherein the controller is further configured to display a user interface, and the trigger signal is received via the user interface, and/ or
wherein the controller is further configured to:
receive feedback associated with blood flow through the blood pump when operated according to the second timing mode; and adjust the delay of the second timing mode based, at least in part, on the feedback, wherein adjusting the delay of the second timing mode preferably comprises increasing the delay.

5. The blood pump system of any one of the preceding claims, wherein the controller is further configured to:
receive feedback associated with blood flow through the blood pump when operated according to the second timing mode; and
control the motor to drive rotation of the rotor according to the first timing mode based, at least in part, on the feedback,
wherein the feedback preferably includes values for one or more ventricular parameters, wherein the one or more ventricular parameters preferably include one or more of ventricular volume, ventricular pressure, or contractility.

6. The blood pump system of any one of the preceding claims, where the controller is further configured to:
control, after a predetermined amount of time following entering the second timing mode, the motor to drive rotation of the rotor according to the first timing mode;
receive feedback associated with blood flow through the blood pump when operated according to the first timing mode; and
control the motor to operate in the first timing mode or the second timing mode based, at least in part, on the feedback.

7. The blood pump system of claim 6, wherein the feedback includes values for one or more ventricular parameters, wherein the one or more ventricular parameters preferably include one or more of ventricular volume, ventricular pressure, or contractility.

8. A controller for a blood pump system, the controller comprising:
at least one hardware processor configured to:
receive a trigger signal indicating low blood flow through a blood pump of the blood pump system when a motor of the blood pump system is operated according to a first timing mode; and
control the motor to operate according to a second timing mode in response to receiving the trigger signal, wherein the second timing mode includes a delay relative to the first timing mode.

9. The controller of claim 8, wherein the first timing mode is configured to drive rotation of a rotor of the blood pump in accordance with each cardiac cycle of a heart of a patient, and/ or
wherein the at least one hardware processor is further configured to detect a suction event associated with operation of the blood pump, and the trigger signal is received in response to detecting the suction event, wherein the trigger signal is preferably received in response to determining that the suction event has persisted for an amount of time that exceeds a threshold amount of time.

10. The controller of claim 8 or 9, wherein the at least one hardware processor is further configured to generate the trigger signal based, at least in part, on one or more ventricular parameters,
wherein the one or more ventricular parameters preferably include one or more of ventricular volume, ventricular pressure, or contractility, and/ or
wherein the at least one hardware processor is preferably further configured to generate the trigger signal based, at least in part, on a current speed of the motor, and/ or
wherein the at least one hardware processor is preferably further configured to receive one or more signals from a device separate from the blood pump system; and determine the one or more ventricular parameters based on the received one or more signals, and/ or
wherein the at least one hardware processor is preferably further configured to receive the one or more ventricular parameters from a device separate from the blood pump system, wherein the device separate from the blood pump system preferably comprises one or more of an impedance catheter, a swan-ganz catheter, an imaging device, an echocardiography device, or an ultrasound patch.

11. The controller of any one of the preceding claims 8 to 10, wherein the second timing mode is configured to drive rotation of a rotor of the blood pump at a multiple of a cardiac cycle of a heart of a patient, and/ or
wherein the second timing mode is configured to drive rotation of a rotor of the blood pump using a timing longer than a cardiac cycle of a heart of a patient, and/ or
wherein the at least one hardware processor is further configured to display a user interface, and the trigger signal is received via the user interface, and/ or
wherein the at least one hardware processor is further configured to receive feedback associated with blood flow through the blood pump when operated according to the second timing mode; and adjust the delay of the second timing mode based, at least in part, on the feedback, wherein adjusting the delay of the second timing mode preferably comprises increasing the delay.

12. The controller of any one of the preceding claims 8 to 11, wherein the at least one hardware processor is further configured to:
receive feedback associated with blood flow through the blood pump when operated according to the second timing mode; and
control the motor to drive rotation of a rotor of the blood pump according to the first timing mode based, at least in part, on the feedback, wherein the feedback preferably includes values for one or more ventricular parameters, wherein the one or more ventricular parameters preferably include one or more of ventricular volume, ventricular pressure, or contractility.

13. The controller of any one of the preceding claims 8 to 12, where the at least one hardware processor is further configured to:
control, after a predetermined amount of time following entering the second timing mode, the motor to drive rotation of a rotor of the blood pump according to the first timing mode;
receive feedback associated with blood flow through the blood pump when operated according to the first timing mode; and
control the motor to operate in the first timing mode or the second timing mode based, at least in part, on the feedback.

14. The controller of claim 13, wherein the feedback includes values for one or more ventricular parameters.

15. The controller of claim 14, wherein the one or more ventricular parameters include one or more of ventricular volume, ventricular pressure, or contractility.
